Europäisches Patentamt

**European Patent Office**

**Office européen des brevets**

(19)

(11) Veröffentlichungsnummer: **0 137 863**

**B1**

(12)

# EUROPÄISCHE PATENTSCHRIFT

(45) Veröffentlichungstag der Patentschrift:
**10.08.88**

(21) Anmeldenummer: **83110280.1**

(22) Anmeldetag: **15.10.83**

(51) Int. Cl.⁴: **A 61 N 1/42**

(54) **Vorrichtung zur Erzeugung eines magnetischen Feldes an Körperteilen.**

(43) Veröffentlichungstag der Anmeldung:
**24.04.85 Patentblatt 85/17**

(45) Bekanntmachung des Hinweises auf die Patenterteilung:
**10.08.88 Patentblatt 88/32**

(84) Benannte Vertragsstaaten:
**AT BE CH DE FR GB IT LI LU NL SE**

(56) Entgegenhaltungen:
**WO-A-84/00305**
**DE-A-2 733 982**
**DE-A-3 041 965**
**DE-A-3 246 128**
**DE-U-6 904 160**
**FR-A-716 954**
**FR-A-1 215 110**
**FR-A-1 457 985**
**FR-A-2 115 074**

(73) Patentinhaber: **Technitron Elektrik AG, Baarer Strasse 59, CH- 6300 Zug (CH)**

(72) Erfinder: **Bucher, Heinz, Turmweg 44, D-7210 Rottweil (DE)**

(74) Vertreter: **Riebling, Peter, Dr.- Ing., Patentanwälte Dr.- Ing., Dipl.- Ing., Ing.(grad.) Günter Riebling Dr.- Ing., Dipl.- Ing. Peter Riebling Rennerle 10 Postfach 3160, D-8990 Lindau (DE)**

EP 0 137 863 B1

## Beschreibung

Die Erfindung betrifft eine Vorrichtung zur Erzeugung eines magnetischen Feldes an Körperteilen durch Verwendung von elastischen, flachen Magnetträgern in Folienform bei einem Unterbett mit Magnetbändern, bei denen in einer Bindesubstanz eingebettete, magnetisierbare Partikel und/oder Einzelmagnete vorhanden sind.

Eine derartige Vorrichtung ist durch die FR-A-2 115 074 bekannt geworden. Eine ähnliche Vorrichtung, die allerdings nicht auf die Anwendung an einem Unterbett mit Magnetbändern Bezug nimmt, beschreibt im übrigen die DE-GM 6 904 160. Dort wird zur Erzeugung eines magnetischen Feldes ein elastischer, flacher Körper in Folienform eingesetzt. Dieser kann einseitig oder beidseitig mit magnetischem Material beschichtet sein. Dieser Magnetträger wird entsprechend der Lehre der erwähnten DE-GM dann an Körperteilen befestigt.

Die bekannte Vorrichtung beruht auf der Erkenntnis, daß durch die Anwendung magnetischer und/oder elektrostatischer Kraftfelder Einfluß auf das Ionengeschehen im menschlichen Körper genommen wird.

Es stößt auf örtliche Schwierigkeiten, wenn man das Bett nach dem magnetischen Kraftfeld ausrichten will. Zu berücksichtigen ist, daß das magnetische Kraftfeld oft nur sehr gestört in die Schlafräume gelangt. So ist es bekannt, daß in Betonbauten durch das eiserne Baustahlgewebe weitgehend eine Abschirmung des magnetischen Feldes erfolgt, da dieses Baustahlgewebe einem faradayischen Käfig entspricht. Auch in Kraftwagen in einer Limousine tritt diese Abschirmung des Erdmagnetfeldes auf.

Hier setzt die Erfindung ein, der die Aufgabe zugrunde liegt, den menschlichen Körper einem gerichteten, magnetischen Kraftfeld auszusetzen, vorzugsweise einem von Norden nach Süden verlaufenden Feld oder umgekehrt.

Die Lösung der Aufgabe nach der Erfindung besteht darin, daß die Magnetträger so angeordnet sind, daß im mittleren Bereich des Unterbettes, d.h. ungefähr entsprechend der Körpermitte eines auf dem Unterbett liegenden Menschen auf der einen, z. B. oberen Seite des Unterbettes, der eine Pol, z. B. der Nordpol, angeordnet ist und demzufolge auf der unteren Seite des Unterbettes sich der andere Pol, z. B. der Südpol, befindet, während im unteren Bereich des Unterbettes, d.h. dort, wo sich ungefähr die Beine befinden würden, umgekehrt auf der Oberseite des Unterbettes der andere Pol, z. B. der Südpol, und dort auf der Unterseite der eine Pol, z. B. der Nordpol, angeordnet ist.

Durch eine derartige Maßnahme wird ein gerichtetes Magnetfeld erzeugt, welches sowohl von Norden nach Süden verläuft, d.h. von der Körpermitte her nach den Beinen, als auch senkrecht zur Auflage, nämlich von oben nach unten den Körper durchdringt. Die Verwendung eines Unterbettes für die Erzeugung dieses magnetischen Feldes gestattet es, daß jeder das Unterbett Benutzende in beliebiger Lage zum Erdfeld liegen kann, trotzdem aber magnetisch "ausgerichtet" schläft. Der Einfluß des vom Unterbett ausgehenden, magnetischen Feldes ist wegen dem geringen Abstand zum daraufliegenden Körper um ein Mehrfaches größer, als der Einfluß des magnetischen Erdfeldes, so daß dieses nicht sich störend auswirken kann, selbst wenn das Unterbett in einer anderen Richtung liegt als das magnetische Erdfeld verläuft.

Die gleiche Vorrichtung wird noch dazu verwandt, daß die Magnetträger bei einem Kopfkissen so angeordnet sind, daß auf der Oberseite des Kopfkissens die gleiche Polung wie auf der Oberseite des zugeordneten Unterbettes der Körpermitte, z. B. der Nordpol, angeordnet ist, und damit die Unterseite den Südpol aufweist.

Das Kopfkissen hat also die gleiche Polung wie das daran anschließende Unterbett. Nur im Beinbereich des Unterbettes ist die entgegengesetzte Polung vorhanden.

Eine bevorzugte Ausführung besteht darin, daß die Magnetträger als dünne, flexible Streifen ausgebildet in einem Tunnelgewebe angeordnet sind.

Durch diese Maßnahme kann man sogar nachträglich Unterbetten mit derartigen Magnetträgern versehen, insbesondere ist aber eine einfache Fertigung möglich, weil man diese Magnetstreifen nur in das Tunnelgewebe einschiebt und dieses Gewebe dann den Magnetstreifen in dieser Lage hält. Man hat es sogar in der Hand, verschiedene Magnetstreifen zu verwenden, die ein mehr oder weniger starkes magnetisches Feld erzeugen.

Derartige Magnetträger sind so ausgebildet, daß auf der einen - z. B. oberen Seite - der eine Pol, z. B. Nordpol, und auf der unteren Seite der andere Pol vorhanden ist.

Wichtig ist, daß bei einem Unterbett der Bereich, in dem ungefähr das Kopfkissen liegt, keine Magnetstreifen vorhanden sind. Diese Ausführung wird man wählen, da man das Kopfkissen auf diesen Bereich wählt, und die Magnetstreifen sich nachteilig beeinflussen würden, wenn sie übereinander mit verschiedener Polung liegen würden.

Die besondere konstruktive Ausgestaltung und die Abmaße sowie Ausbildung des Magnetstreifens sind weiter erfinderische Merkmale, sind aber in der Zeichnungsbeschreibung näher erläutert.

Wesentlich ist noch, daß die Magnetstreifen als Bindesubstanz für die eingebetteten, magnetischen Partikel ca. 5 % Kautschul aufweisen.

Man kann diese Beschichtung mit Partikel ein- oder beidseitig durchführen. Es handelt sich um ein bekanntes Verfahren, das - wenn es einem starken, magnetischen Feld ausgesetzt wird - dann diese Partikel magnetisiert werden und aufgrund ihrer hohen Koerzitivkraft dann die magnetische Polung beibehalten.

Selbstverständlich ist es möglich, daß zusätzlich zu den Magnetstreifen noch weitere wärmende Füllungen sich im Unterbett befinden und/oder im Kopfkissen.

Statt Permanentmagneten können auch Elektromagnete verwandt werden und man kann, wenn man Elektromagnete verwendet, auch das Unterbett mit elektrischen Heizwiderständen versehen, so daß eine Wärmewirkung zusätzlich vorhanden ist.

Ein Ausführungsbeispiel der Erfindung ist in der Zeichnung dargestellt. Dabei gehen aus der Zeichnung und der Beschreibung weitere Erfindungsmerkmale hervor.

Figur 1    zeigt schematisch ein Unterbett in der Draufsicht;
Figur 2    zeigt ein diesem Unterbett zugeordnetes Kopfkissen in der Draufsicht;
Figur 3    ist ein Schnitt längs der Linie I - I in schematischer Darstellung in der Figur 1;
Figur 4    ist ein Schnitt längs der Linie II - II der Figur 2 in schematischer Ansicht;
Figur 5    zeigt eine andere Ausführung des Magnetstreifens;
Figur 6    zeigt die Möglichkeit der Verwendung von Elektromagneten zur Erzeugung des magnetischen Feldes;

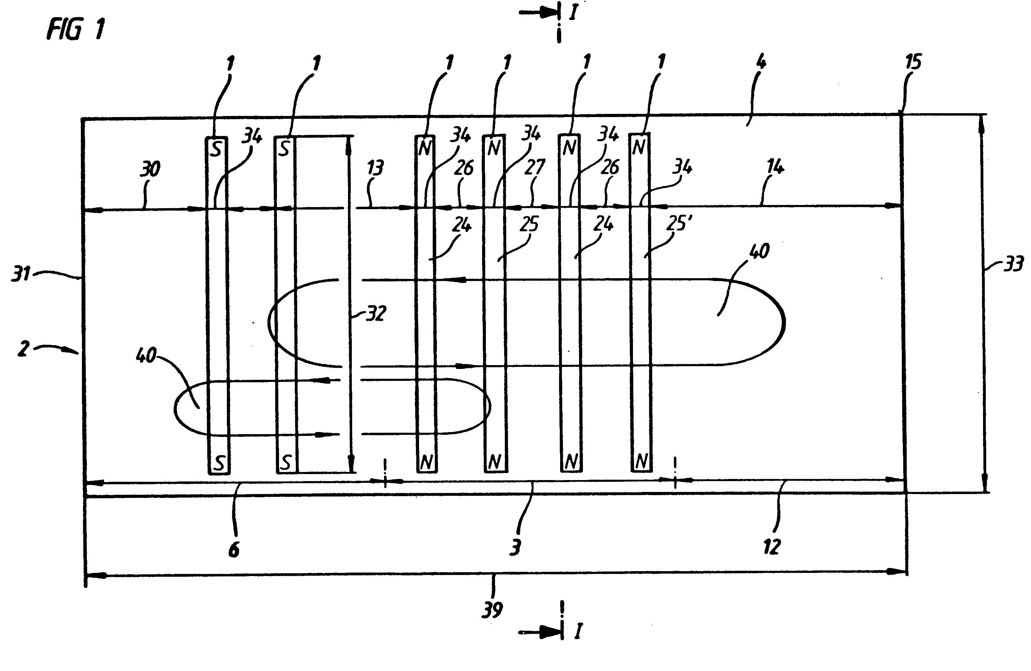

In der Figur 1 sind Magnetträger 1 dargestellt, die im Unterbett 2 angeordnet sind. Diese Magnetträger 1 laufen zueinander parallel. In einem mittleren Bereich 3 sind die Magnetstreifen so gepolt, daß deren obere Seite 4 einen Nordpol (N) und deren untere Seite einen Südpol (S) aufweist. (Vergl. auch Figur 3).

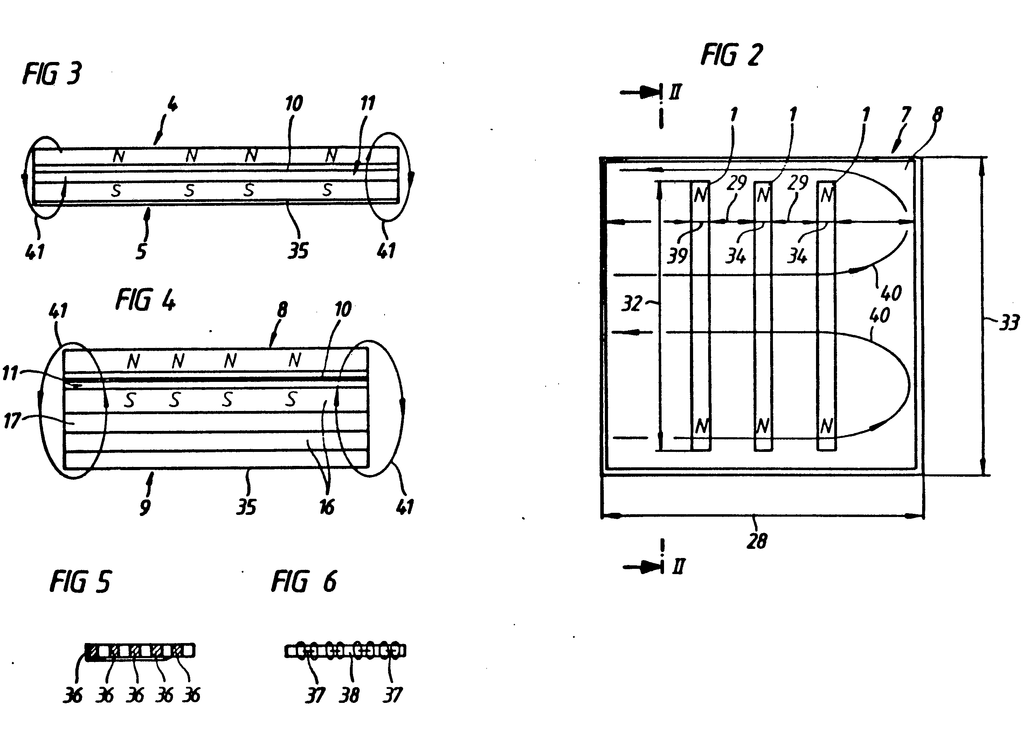

Entsprechend ist das Kopfkissen 7 ebenfalls mit Magnetträgern 1 versehen, die ebenso - wie beim Unterbett 2 - parallel zueinander verlaufend, parallel zur oberen Randkante 15 des Unterbettes bzw. der entsprechenden Kante des Kopfkissens, verlaufen. Die obere Seite 8 des Kopfkissens weist dann analog im Bereich 3 im Unterbett 2 Nordpole auf und dementsprechend die Unterseite 9 Südpole. (Vergl. hierzu auch Figur 4).

Die Magnetstreifen 1 sind als flexible Streifen 10 ausgebildet, die in ein Tunnelgewebe 11 eingeschoben werden, welches sowohl im Unterbett als auch im Kopfkissen vorhanden ist. Der Bereich 12 des Unterbettes, auf den das Kopfkissen zu liegen kommt, besitzt keine Magnetstreifen 1. Die Abmaße des Unterbettes sind dann üblicherweise so gewählt, daß die Schmalseite 33 z. B. 90 cm ist und die Längsseite 39 entsprechend 200 cm ist.

Das Kopfkissen hat dann im vorliegenden Fall eine quadratische Form, wobei die Breite 28 des Kopfkissens gleich der anderen Seite 33 des Kopfkissens ist und diese Abmaße 75 cm betragen. Bei der Ausführungsform sind die Streifen so angeordnet, daß der Abstand 13 zwischen den Streifen des Beinbereiches und der Körpermitte, d.h. untere Bereich 6 und mittlere Bereich 3, ungefähr die Hälfte von dem Abstand 14 ist, nämlich von der oberen Randkante 15 des Unterbettes bis zum ersten Magnetstreifen 25'.

Beim Kopfkissen ist noch zusätzlich eine Baumwollhülle 16 vorhanden, in der sich eine Kopfkissenfüllung 17, z. B. aus Hollofill, befindet.

Die Magnetstreifen 24, 25, 25' sind im mittleren Bereich 3 so angeordnet, daß ein kürzerer Abstand 26 zwischen diesen Paaren vorhanden ist, wobei das eine Paar von den Streifen 24, 25 und das andere Paar von den Streifen 24, 25' gebildet wird. Dieser Abstand 26 ist kürzer als der Abstand 27 zwischen den Paaren. Beim Kopfkissen sind die Streifen mit einem gleichmäßigen Abstand 29 voneinander angeordnet; im übrigen ist der Abstand 30 im unteren Bereich 6, der der unteren Randkante 31 am nächsten liegende Magnetstreifen aufweist, ungefähr genauso groß, wie der Abstand 13 zwischen dem ersten Streifen des Magnetstreifens des unteren Bereiches 6 und dem letzten Streifen des mittleren Bereiches 3.

Die Länge 32 der Magnetstreifen ist ca. 10 % kleiner als die Länge 33 der Schmalseiten. Die Breite 34 der Magnetstreifen beträgt im Ausführungsbeispiel ca. 5 cm. Bei den Figuren 3 und 4 ist noch die Möglichkeit vorgesehen, zusätzliche Folien 35 aus unmagnetischem Material vorzusehen.

Eine weitere Möglichkeit der Ausbildung der Magnetstreifen 1 ist der in Figur 5 dargestellte Magnetstreifen, bei dem in gleichmäßigem Abstand immer unmagnetische Teile 36 vorhanden sind. Derartige Streifen könnte man ggf. anwenden, um die Herstellung dieser an sich teuren Magnetstreifen zu verringern.

Insbesondere, wenn man dieses Magnetfeld in eine Heizdecke einarbeiten will und man sowieso elektrische Spannung zur Verfügung hat, kann man flache Elektromagnete 37 einarbeiten, die die Figur 6 zeigt, von Windungen umschlossen sind und in ein Kunststoffband 38 eingebettet sind.

Schematisch ist in den Figuren angedeutet, daß ein von Norden nach Süden verlaufendes magnetisches Feld 40 vorhanden ist, welches sich zwischen den Nordpolen des mittleren Bereiches 3 und dem Kopfkissen ausbildet. Zusätzlich hierzu ist ein weiteres magnetisches Feld 41 vorhanden, welches sich senkrecht zur Oberfläche von Unterbett und Kopfkissen ausbildet, wobei die magnetischen Kraftlinien aus dem Nordpol austreten und beim Südpol wieder eintreten, wie das schematisch in den Figuren 3 und 4 dargestellt ist.

**Patentansprüche**

1. Vorrichtung zur Erzeugung eines magnetischen Feldes an Körperteilen durch Verwendung von elastischen, flachen Magnetträgern (1) in Folienform bei einem

Unterbett (2) mit Magnetbändern, bei denen in einer Bindesubstanz eingebettete, magnetisierbare Partikel und/oder Einzelmagnete vorhanden sind, dadurch gekennzeichnet, daß die Magnetträger (1) so angeordnet sind, daß im mittleren Bereich (3) des Unterbettes (2), d.h. ungefähr entsprechend der Körpermitte eines auf dem Unterbett liegenden Menschen, auf der einen, z. B. oberen Seite (4) des Unterbettes (2), der eine Pol (N) angeordnet ist, und demzufolge auf der unteren Seite (5) des Unterbettes (2) sich der andere Pol (5) befindet, während im unteren Bereich (6) des Unterbettes (2), d.h. dort, wo sich ungefähr die Beine befinden würden, umgekehrt auf der Oberseite (4) des Unterbettes (2) der andere Pol (5) und dort auf der Unterseite (5) der eine Pol (N) angeordnet ist.

2. Vorrichtung zur Verwendung mit der Vorrichtung nach Patentanspruch 1, dadurch gekennzeichnet, daß weitere Magnetträger (1) bei einem Kopfkissen (7) so angeordnet sind, daß auf der Oberseite (8) des Kopfkissens (7) die gleiche Polung (N) wie auf der Oberseite (4) des zugeordneten Unterbettes (2) im mittleren Bereich (3) des Unterbettes angeordnet ist und damit die Unterseite (9) des Kopfkissens (7) den anderen Pol (S) aufweist.

3. Vorrichtung nach Anspruch 1 oder 2, dadurch gekennzeichnet, daß die Magnetträger (1) als dünne, flexible Streifen (10) ausgebildet in einem Tunnelgewebe (11) angeordnet sind.

4. Vorrichtung nach Anspruch 1 bis 3, dadurch gekennzeichnet, daß die Magnetträger (1) auf der einen, z. B. oberen Seite, den einen Pol, z. B. Nordpol (N), und auf der unteren Seite den anderen Pol (S) aufweisen.

5. Vorrichtung nach Anspruch 4, dadurch gekennzeichnet, daß bei einem Unterbett (2) der Bereich (12), in dem ungefähr das Kopfkissen (7) aufliegt, keine Magnetstreifen (1) vorhanden sind.

6. Vorrichtung nach Anspruch 1 bis 3, dadurch gekennzeichnet, daß bei einem Unterbett (2) der untere Bereich (6) mit entgegengesetzter Polung, der den Beinen zugeordnet ist, ungefähr den halben Abstand (13) vom magnetischen Bereich (3), der der Körpermitte zugeordnet ist, aufweist, wie dessen Abstand (14) zur oberen Randkante des Unterbettes (2) beträgt.

7. Vorrichtung nach Anspruch 1 bis 6, dadurch gekennzeichnet, daß die magnetisch einander zugeordneten Seiten (4, 8) des Unterbettes (2) und des Kopfkissens (7) gleiche Farbgebung aufweisen, d.h. die Oberseite (4, 8) eine unterschiedliche Farbgebung gegenüber den Unterseiten (5, 9) besitzt.

8. Vorrichtung nach Anspruch 1 bis 7, dadurch gekennzeichnet, daß die Oberseite (4, 8) von einem weißen und die Unterseite (5, 9) von einem braunen Wirkpelz gebildet ist.

9. Vorrichtung nach Anspruch 1, dadurch gekennzeichnet, daß beim Unterbett (2) zwischen dem weißen und braunen Wirkpelz ein Tunnelgewebe (11) vorhanden ist, welches Taschen für die Aufnahme der Magnetstreifen (1) aufweist.

10. Vorrichtung nach Anspruch 2, dadurch gekennzeichnet, daß beim Kopfkissen (7) zwischen Tunnelgewebe (11) und Magnetstreifen (1 bzw. 10) und dem braunen Wirkpelz in einer Baumwollhülle (16) die Kopfkissenfüllung (17), z. B. Hollofill, vorhanden ist.

11. Vorrichtung nach Anspruch 1, dadurch gekennzeichnet, daß die Magnetstreifen (1) unterschiedliche magnetische Feldstärken aufweisen.

12. Vorrichtung nach Anspruch 1, dadurch gekennzeichnet, daß die Magnetstreifen (1) als Bindesubstanz für die eingebetteten, magnetisierbaren Partikel ca. 5 % Kautschuk aufweisen.

13. Vorrichtung nach Anspruch 1, dadurch gekennzeichnet, daß sich die Magnetstreifen (1) im mittleren Querschnitt des Unterbettes (2) befinden, welches weitere, wärmende Füllungen, z. B. aus Schafschurwollflies, aufweist.

14. Vorrichtung nach Anspruch 1, dadurch gekennzeichnet, daß das Unterbett (2) und/oder Kopfkissen (7) aus Naturfasern besteht.

15. Vorrichtung nach Anspruch 1, dadurch gekennzeichnet, daß das Unterbett (2) und/oder Kopfkissen (7) ganz oder teilweise aus Kunststoff-Fasern besteht.

16. Vorrichtung nach Anspruch 2, dadurch gekennzeichnet, daß das Kopfkissen (7) quadratisch ist.

17. Vorrichtung nach Anspruch 1, dadurch gekennzeichnet, daß im mittleren Bereich (3), d.h. in der Körpermitte, vier Magnetstreifen (1) vorhanden sind.

18. Vorrichtung nach Anspruch 17, dadurch gekennzeichnet, daß je zwei nebeneinanderliegende, der Körpermitte zugeordnete, Magnetstreifen (24, 25, 25') als Paar einen kürzeren Abstand (26) voneinander haben als die Paare (24, 25, 25') von sich den Abstand (27) aufweisen.

19. Vorrichtung nach Anspruch 1, dadurch gekennzeichnet, daß der dem Kopfteil (12) am nächsten liegende Magnetstreifen (25') einen so großen Abstand (14) von der kopfseitigen, oberen Randkante (15) des Unterbettes (2) hat, wie ungefähr die Breite (28) des Kopfkissens (7) ist.

20. Vorrichtung nach Anspruch 19, dadurch gekennzeichnet, daß im Kopfkissen (7) drei Magnetstreifen (1) angeordnet sind, deren Abstand (29) voneinander ungefähr den Abstand (26) der den mittleren Bereich (3) zugeordneten Magnetstreifen (1) entspricht.

21. Vorrichtung nach Anspruch 1, dadurch gekennzeichnet, daß die dem unteren Bereich (6) zugeordneten Magnetstreifen (1) umgekehrte Polung wie die Magnetstreifen (1) des mittleren Bereiches (3) besitzen und gleichen Abstand (30) zur beinseitigen, unteren Randkante (31) des Unterbettes (2) und zum nächstliegenden Magnetstreifen des mittleren Bereiches (3) aufweisen.

22. Vorrichtung nach Anspruch 1, dadurch gekennzeichnet, daß die Länge (32) der Magnetstreifen (1) im Unterteil (2) und Kopfkissen (7) ca. 10 - 12 % kürzer ist als die Länge der Schmalseiten des Unterbettes (2) bzw. der hierzu parallel liegenden Seite des Kopfkissens (7).

23. Vorrichtung nach Anspruch 1 bis 22, dadurch gekennzeichnet, daß die Dicke der Magnetstreifen (1) ca. 1 mm und deren Breite (34) ca. 5 cm ist.

24. Vorrichtung nach Anspruch 1, dadurch gekennzeichnet, daß innerhalb der Magnetstreifen (1) in gleichmäßigen Abständen unmagnetische Teile (36), z. B. aus Kunststoff, angeordnet sind.

25. Vorrichtung nach Anspruch 1, dadurch gekennzeichnet, daß auf einem unmagnetischen Streifen im Abstand Magnetfolienstücke und/oder Permanentmagnete aufgebracht sind.

26. Vorrichtung nach Anspruch 1, dadurch gekennzeichnet, daß Elektromagnete statt der Permanentmagnete vorhanden sind.

27. Vorrichtung nach Anspruch 26, dadurch gekennzeichnet, daß die Elektro-Magnetwicklungen (37) mit ihren Polkernen in Kunststoffbändern (38) eingebettet sind.

**Claims**

1. Device for generating a magnetic field on body sections by the use of elastic, flat magnet carriers (1) in foil form on a mattress (2) comprising magnet strips in which are present magnetisable particles and/or individual magnets embedded in a bonding substance, characterised in that the magnet carriers (1) are so arranged that in the central area (3) of the mattress (2), i.e. corresponding approximately to the middle of the body of a person lying on the mattress, the one pole (N) is situated on the one side, e.g. the upper side (4), of the bed base (2), and that the other pole (S) is consequently situated on the underside (5) of the mattress (2), whereas in the lower region (6) of the mattress (2), i.e. approximately where the legs would be positioned, the other pole (S) is conversely placed on the upper side (4) of the mattress (2) and the first pole (N) is placed there on the underside (5).

2. Device for use with the device according to claim 1, characterised in that other magnet carriers (1) are so arranged in the case of a pillow (7) that the same polarity (N) as that situated on the upper side (4) of the associated mattress (2) in the central area (3) of the mattress is arranged on the upper side (8) of the pillow (7), and that the underside (9) of the pillow (7) thus has the other pole (S).

3. Device according to claim 1 or 2, characterised in that the magnet carriers (1) are formed as thin flexible strips (10), arranged in a tunnel fabric (11).

4. Device according to claims 1 to 3, characterised in that the magnet carriers (1) have the one pole, e.g. the North pole (N) on the one side, e.g. the upper side, and the other pole (S) on the lower side.

5. Device according to claim 4, characterised in that in the case of a mattress (2) no magnet strips (1) are present in approximately the area (12) on which the pillow (7) is laid.

6. Device according to claims 1 to 3, characterised in that in the case of a mattress (2), the lower section (6) of opposite polarity, which is allocated to the legs, is positioned at approximately half the distance (13) from the magnetic area (3) allocated to the middle of the body, that is half the distance (14) of the latter from the top peripheral edge of the mattress (2).

7. Device according to claims 1 to 6, characterised in that the sides (4, 8) of the mattress (2) and of the pillow (7) which are magnetically associated one with another, have the same colouration, i.e. the top side (4, 8) has a different colouration as compared to the undersides (5, 9).

8. Device according to claims 1 to 7, characterised in that the top side (4, 8) is formed by a white raised knitted fabric, and the underside (5, 9) by a brown raised knitted fabric.

9. Device according to claim 1, characterised in that a so-called tunnel fabric (11) which comprises pockets for reception of the magnet strips (1) is present in the mattress (2) between the white and brown raised knitted fabrics.

10. Device according to claim 2, characterised in that the pillow filling (17), e.g. hollow-fill, is present in the pillow within a cotton sheath (16) between the tunnel fabric (11) and magnet strips (1 and 10 respectively) and the brown raised knitted fabric.

11. Device according to claim 1, characterised in that the magnet strips (1) have different magnetic field strengths.

12. Device according to claim 1, characterised in that the magnet strips incorporate approximately 5 % of rubber as a bonding substance for the embedded magnetisable particles.

13. Device according to claim 1, characterised in that the magnet strips (1) are situated in the central cross-section of the mattress (2) which contains other thermal fillings, e.g. of clipped wool fleece.

14. Device according to claim 1, characterised in that the mattress (2) and/or the pillow (7) comprises natural fibres.

15. Device according to claim 1, characterised in that the mattress (2) and/or the pillow (7) consists wholly or partly of plastics material fibres.

16. Device according to claim 1, characterised in that the pillow (7) is square.

17. Device according to claim 1, characterised in that four magnet strips (1) are present in the central area (3), i.e. at the middle of the body.

18. Device according to claim 17, characterised in that each two magnet strips (24, 25, 25') which are adjacently situated and allocated to the centre of the body have a shorter spacing (26) as a pair from one another than the spacing (27) between the pairs (24, 25, 25').

19. Device according to claim 1, characterised in that the magnet strip (25') lying closest to the head section (12) has a spacing (14) from the upper marginal edge (15) at the head end of the mattress (2) which is approximately as great as the width (28) of the pillow (7)

20. Device according to claim 19, characterised in that three magnet strips (1) are situated in the pillow (7), their mutual spacing (29) corresponding approximately to the spacing (26) of the magnet strips (1) allocated to the central area (3).

21. Device according to claim 1, characterised in that the magnet strips (1) allocated to the lower section (6) are reversed in polarity compared to the magnet strips (1) of the central area (3) and have the same spacing (30) from the bottom marginal foot-end edge (31) of the mattress (2) and from the nearest magnet strip of the central area (3).

22. Device according to claim 1, characterised in that the length (32) of the magnetic strips (1) in the under part (2) and the pillow (7) is approximately 10 - 12 % shorter than the length of the narrow sides of the mattress (2), or of the pillow (7) extending parallel thereto.

23. Device according to claims 1 to 22, characterised in that the thickness of the magnet strips (1) is approximately 1 mm, and their width (34) is approximately 5 cm.

24. Device according to claim 1, characterised in that non-magnetic parts (36), e.g. of plastics material, are arranged at regular intervals within the magnet strips (1).

25. Device according to claim 1, characterised in that magnetic foil elements and/or permanent magnets are applied at intervals on a non-magnetic strip.

26. Device according to claim 1, characterised in that electromagnets are present instead of the permanent magnets.

27. Device according to claim 26, characterised in that the electromagnet windings (37) have their pole cores embedded in plastics material tapes (38).

**Revendications**

1. Dispositif pour produire un champ magnétique sur des parties du corps par utilisation de supports magnétiques plats, élastiques (1) présentés sous la forme de pellicules, dans le cas d'un matelas (2) munis de bandes magnétiques, dans lesquelles sont présentes des particules magnétisables et/ou des aimants individuels, noyés dans une substance liante, caractérisé en ce que les supports magnétiques (1) sont agencés de telle manière que, dans la région médiane (3) du matelas (2), c'est-à-dire à peu près au niveau du milieu du corps d'un être humain couché sur le matelas, un premier pôle (N) se trouve sur une face, par exemple, sur la face supérieure (4) du matelas (2), et, par conséquent le second pôle (5) se trouve sur la face inférieure (5) du matelas (2) tandis que, dans la région inférieure (6) du matelas (2), c'est-à dire, à l'endroit ou se trouverait à peu près les jambes, le second pôle (S) se trouve au contraire sur la face supérieure (4) du matelas (2), et le premier pôle (N) se trouve sur sa face inférieure (5).

2. Dispositif destiné à être utilisé avec le dispositif selon la revendication 1, caractérisé en ce que d'autres supports magnétiques (1) sont disposés sur un oreiller (7) de telle manière que, sur la face supérieure (8) de l'oreiller (7), se trouve le même pôle (N) que sur la face supérieure (4) du matelas (2) correspondant, dans la région médiane (3) du matelas, et que la face inférieure (9) de l'oreiller (7) présente donc le second pôle (S).

3. Dispositif selon la revendication 1 ou 2, caractérisé en ce que les supports magnétiques (1) sont réalisés sous la forme de bandes minces et flexibles (10) et sont disposés dans un tissu housse (11).

4. Dispositif selon l'une des revendications 1 à 3, caractérisé en ce que les supports magnétiques (1) présentent, sur une face, par exemple la face supérieure, un premier pôle, par exemple le pôle Nord (N) et sur la face inférieure, le second pôle (S).

5. Dispositif selon la revendication 4, caractérisé en ce que, dans un matelas (2), la région (12), dans laquelle se trouve approximativement l'oreiller (7) , ne comporte pas de bandes magnétiques (1).

6. Dispositif selon l'une des revendications 1 à 3, caractérisé en ce que, dans un matelas (2), la région inférieure (6) qui est associée aux jambes est éloignée de la région magnétique (3), de polarité opposée, associée au milieu du corps, d'une distance (13) à peu près égale à la moitié de la distance (14) qui sépare cette dernière région du bord supérieur du matelas (2).

7. Dispositif selon l'une des revendications 1 à 6, caractérisé en ce que les faces (4, 8) du matelas (2) et de l'oreiller (7) qui sont accordées magnétiquement l'une à l'autre présentent la même coloration, c'est-à-dire que la face supérieure (4, 8) possède une coloration différente de celle des faces inférieures (5, 9).

8. Dispositif selon l'une des revendications 1 à 7, caractérisé en ce que la face supérieure (4, 8) est formée d'une fourrure tricotée blanche et la face inférieure (5, 9) d'une fourrure tricotée marron.

9. Dispositif selon la revendication 1, caractérisé en ce que, dans le matelas (2), est prévu, entre la fourrure tricotée blanche et la fourrure tricotée marron, un tissu housse (11) qui présente des poches destinées à recevoir les bandes magnétiques (1).

10. Dispositif selon la revendication 2, caractérisé en ce que, dans l'oreiller (7), le rembourrage d'oreiller (17), par exemple, en hollofill, est prévu entre le tissu housse (11) et les bandes magnétiques (1 et 10 respectivement) , et la fourrure tricotée marron, dans une enveloppe en coton (16).

11. Dispositif selon la revendication 1, caractérisé en ce que les bandes magnétiques (1) présentent des intensités de champ magnétique différentes.

12. Dispositif selon la revendication 1, caractérisé en ce que les bandes magnétiques (1) présentent, comme substance liante pour les particules magnétisables noyées, environ 5 % de caoutchouc.

13. Dispositif selon la revendication 1, caractérisé en ce que les bandes magnétiques (1) se trouvent au milieu de la section transversale du matelas (2), qui présente d'autres rembourrages chauds, par exemple une nappe de laine naturelle.

14. Dispositif selon la revendication 1, caractérisé en ce que le matelas (2) et/ou l'oreiller (7) sont composés de fibres naturelles.

15. Dispositif selon la revendication 1, caractérisé en ce que le matelas (2) et/ou l'oreiller (7) sont composés entièrement ou partiellement de fibres synthétiques.

16. Dispositif selon la revendication 2, caractérisé en ce que l'oreiller (7) est de forme carrée.

17. Dispositif selon la revendication 1, caractérisé en ce que quatre bandes magnétiques (1) sont prévues dans la région médiane (3), c'est-à-dire au milieu du corps.

18. Dispositif selon la revendication 17, caractérisé en ce que les bandes magnétiques (24, 25, 25') associées au milieu du corps sont regroupées en deux paires dans chacune desquelles les deux bandes sont juxtaposées avec un écartement mutuel (26) inférieur à la distance (27) qui sépare les paires (24, 25, 25').

19. Dispositif selon la revendication 1, caractérisé en ce que la bande magnétique (25'), qui est la plus proche de la partie de tête (12), est séparée du bord supérieur (15), côté tête, du matelas (2) d'une distance (14) qui est à peu près égale à largeur (28) de l'oreiller (7).

20. Dispositif selon la revendication 19, caractérisé en ce que, dans l'oreiller (7), sont disposées trois bandes magnétiques (1) dont l'écartement mutuel (29) correspond à peu près à la distance d'écartement (26) entre les bandes magnétiques (1) associées à la région médiane (3).

21. Dispositif selon la revendication 1, caractérisé en ce que les bandes magnétiques (1) associées à la région inférieure (6) possèdent une polarité inverse de celle des bandes magnétiques (1) de la région médiane (3) et présentent la même distance d'écartement (30) par rapport au bord inférieur (31), côté jambes, du matelas (2), que par rapport à la bande magnétique immédiatement adjacente de la région médiane (3).

22. Dispositif selon la revendication 1, caractérisé en ce que la longueur (32) des bandes magnétiques (1) situées dans l'élément inférieur (2) et dans l'oreiller (7) est inférieure d'environ 10 à 12 % à la longueur des petits côtés du matelas (2) et, respectivement, à la longueur du côté de l'oreiller (7) qui est parallèle aux petits côtés du matelas.

23. Dispositif selon l'une des revendications 1 à 22, caractérisé en ce que l'épaisseur des bandes magnéques (1) est d'environ 1 mm et leur largeur (34) d'environ 5 cm.

24. Dispositif selon la revendication 1, caractérisé en ce que, des éléments amagnétiques (36), par exemple en matière plastique, sont disposés à l'intérieur des bandes magnétiques (1), avec des intervalles égaux entre eux.

25. Dispositif selon la revendication 1, caractérisé en ce que, des morceaux de pellicule magnétique et/ou des aimants permanents sont disposés sur une bande amagnétique, à distance les uns des autres.

26. Dispositif selon la revendication 1, caractérisé en ce qu'il comprend des electro-aimants à la place des aimants permanents.

27. Dispositif selon la revendication 26, caractérisé en ce que les bobines (37) des électro-aimants sont noyées, avec leur noyau polaire, dans des bandes de matière plastique (38).

FIG 1

FIG 3

N  N  N  N

S  S  S  S

4   10   11

41   5   35   41

FIG 4

41   8   10

N  N  N  N

11

S  S  S  S

17

3

9   35   16   41

FIG 5

36  36  36  36  36

FIG 6

37  38  37

FIG 2

II

1   1   1   7   8

N   29   N   29   N

39   34   34

32   40

40

33

N   N   N

28

II

0 137 863